# EUROPÄISCHE PATENTSCHRIFT

(11) **EP 2 903 842 B1**
(45) Veröffentlichungstag und Bekanntmachung des Hinweises auf die Patenterteilung: **26.02.2020**
(21) Anmeldenummer: 13786431.0
(22) Anmeldetag: 02.10.2013
(51) Int. Cl.: B60H 3/00, B60H 3/06, F24F 3/16, B01D 46/00, B01D 46/10

(54) **SYSTEM ZUR EINBRINGUNG VON DUFTSTOFFEN IN DEN INNENRAUM EINES FAHRZEUGS**
SYSTEM FOR INTRODUCING FRAGRANCES INTO THE INTERIOR OF A VEHICLE
SYSTÈME POUR INTRODUIRE DES MATIERES ODORIFERANTES DANS L'HABITACLE D'UN VEHICULE

(30) Priorität: 02.10.2012 DE 102012019332
(43) Veröffentlichungstag der Anmeldung: 12.08.2015
(73) Patentinhaber: Bayerische Motoren Werke Aktiengesellschaft, 80809 München (DE)
(72) Erfinder: MARSCHALL, Ursula, 48147 Münster (DE); ERGEN, Erhan, 80999 München (DE); MENNE, Andreas, 58730 Fröndenberg (DE)
(86) Internationale Anmeldenummer: PCT/EP2013/070538
(87) Internationale Veröffentlichungsnummer: WO 2014/053539

(56) Entgegenhaltungen:
- EP-A2- 1 721 622
- FR-A1- 2 872 455
- FR-A1- 2 920 312
- KR-B1- 101 172 239
- US-A- 4 563 333
- US-A- 5 087 273

## Beschreibung

Die Erfindung betrifft ein System zur Einbringung von Duftstoffen in den Innenraum eines Fahrzeugs über das Lüftungssystem mit einer Kombination aus einer Duftkartusche und einem Luftfilter, wobei die Duftkartusche wenigstens eine Lufteintrittsöffnung und wenigstens eine Luftaustrittsöffnung aufweist, sowie einen Raum mit einem den Duftstoff abgebenden Substrat, wobei die Duftkartusche am Luftfilter so verankert ist, dass sie bei aktiviertem Lüftungssystem von Luft durchströmt werden kann.

Das "Beduften" von Fahrzeugen ist insbesondere bei zum Wiederverkauf stehenden Gebrauchtwagen allgemein üblich. In der Regel wird hierzu ein Spray eingesetzt, das den Interessenten und Käufer bei der Besichtigung des Fahrzeugs und in den ersten Tagen bzw. Wochen einen frisch und neuwertig riechenden Innenraum präsentiert. Zum gleichen Zweck sind auch Belüftungseinrichtungen bekannt geworden, mit deren Hilfe ein Duftstoff in den Innenraum eines Kraftfahrzeugs abgegeben werden kann. Beispielsweise sei auf die DE 10 2004 017 467 A1 und die DE 102 49 305 A1 hingewiesen.

Die bekannten Belüftungseinrichtungen sind in erster Linie für die Auffrischung von Gebrauchtwagen bestimmt. Neuwagen haben herstellungs- und ausstattungsbedingt einen "frischen" Duft, der sich über eine gewisse Zeit hält. Es gibt jedoch Überlegungen, auch Neuwagen mit einem Beduftungssystem auszustatten, einmal, um in den Innenraum hineingetragenen oder von außen über das Lüftungssystem eindringenden Gerüchen entgegenzuwirken, zum anderen aber auch, um im Rahmen einer "Corporate Identity" dem Fahrzeug einen charakteristischen, für den Hersteller oder (bei Mietwagenfirmen) Betreiber charakteristischen Duft zu verleihen.

Aus der US 5,087,273 A ist ein System zur Einbringung von Duftstoffen in den Innenraum eines Fahrzeugs gemäß Oberbegriff des Anspruchs 1 bekannt.

Die bislang bekannt gewordenen Beduftungssysteme sind entweder aufwendig oder werden unabhängig vom Belüftungssystem aktiv. Im einen Fall sind sie kostenträchtig, im anderen wenig steuerbar und schnell verschöpft, weil sich die Abgabe des Duftstoffs nicht unterbrechen lässt.

Aufgabe der Erfindung ist es, ein Beduftungssystem bereitzustellen, das einfach und kostengünstig Duftstoffe über das Lüftungssystem in den Innenraum eines Fahrzeugs abgeben kann, nur aktiv ist, wenn das Fahrzeug in Gebrauch bzw. in Bewegung ist und im Rahmen normaler Wartungsintervalle erneuert werden kann.

Diese Aufgabe wird mit einem System der eingangs genannten Art gelöst, bei dem die Duftkartusche einen oder mehrere Schieber oder Klappen aufweist, die mit einem Motor gekoppelt sind und die die Öffnungen für den Lufteintritt und/oder Luftaustritt verschließen bzw. freigeben.

Das erfindungsgemäße System kann in praktisch allen Fahrzeugen eingesetzt werden, die über ein Lüftungssystem und einen Luftfilter für das Lüftungssystem ausgestattet sind. Das Lüftungssystem ist von den Benutzern eines Fahrzeugs eingeschränkt steuerbar, so dass es bei aktivierter Lüftung einen konstanten Frischluftstrom über den Lüftungsfilter in den Innenraum des Fahrzeugs transportiert. Die Kombination der Duftkartusche mit den Lüftungsfilter stellt sicher, dass die in der Duftkartusche enthaltenen Duftstoffe mit der Frischluft in den Innenraum des Fahrzeugs gelangen.

Unter Fahrzeug werden geschlossene Fahrzeuge wie Personenkraftwagen, Lastkraftwagen, Autobusse, aber auch Züge und Straßenbahnen verstanden, also Fahrzeuge, die über ein Belüftungssystem verfügen.

Die erfindungsgemäße Duftkartusche weist wenigstens eine Lufteintrittsöffnung und wenigstens eine Luftaustrittsöffnung auf, sowie einen dazwischen angeordneten Raum, der das den Duftstoff abgebende Substrat enthält. Als Substrat wird vorzugsweise ein poröses Granulat verwandt, das mit dem Duftstoff beladen ist. Solche Granulate sind mit einer Vielzahl von Duftstoffen kommerziell erhältlich.

Im erfindungsgemäßen System ist die Duftkartusche fest am Luftfilter des Lüftungssystems verankert. Hierdurch ist sichergestellt, dass sich zwischen der Duftkartusche und dem Lüftungsfilter keine Ablagerungen bilden. Ist die Duftkartusche dem Luftfilter nachgeschaltet, was die bevorzugte Anordnung ist, tritt die gefilterte Luft unmittelbar in die Duftkartusche ein und nimmt dort den Duftstoff auf.

Die feste Verankerung der Duftkartusche erlaubt zum einen den gleichzeitigen und einfachen Austausch von Luftfilter und Duftkartusche bei beispielsweise einer Inspektion des Fahrzeugs, verhindert aber zum anderen, dass die Duftkartusche im Lüftungskanal umherrutscht, in Lüftungskanäle abdriftet oder in eine Position gerät, wo sie nicht mehr vom Luftstrom durchströmt wird. Diese Verankerung kann beispielsweise durch Steckverbinder erfolgen, beispielsweise Stege, ggf. mit Widerhaken, mit denen die Kartusche am Filterelement festgesteckt wird.

Es versteht sich, dass die Duftkartusche nur einen kleinen Teil des Filters abdeckt. Zum Beduften eines Fahrzeugs ist nur eine beschränkte Menge Duftstoff notwendig. Hierzu reicht es beispielsweise aus, dass in einem Faltenfilter herkömmlicher Bauart eine der Filterfalten mit einer solchen Duftkartusche ausgestattet wird. Faltenfilter sind in Kraftfahrzeugen üblich und stellen eine bevorzugte Filterform für erfindungsgemäße Zwecke dar.

Die erfindungsgemäß eingesetzte Duftkartusche ist vorzugsweise formschlüssig mit dem Filter verbunden, beispielsweise über die oben beschriebene Steckverbindung. Formschlüssig heißt in diesem Fall, dass sich das Filterblatt des Filters, insbesondere das Dreieck einer Filterfalte, an die Duftkartusche anschmiegt. Der Luftstrom, der gefiltert aus dem Filter austritt, kann dann über die gesamte Kontaktfläche und die dahinterliegenden Öffnungen in die Duftkartusche eintreten, das den Duftstoff abgebende Substrat durchströmen und mit dem Duftstoff beladen aus der Kartusche wieder austreten. Auf diese Art und Weise entsteht eine Art Zwangsführung des Luftstroms durch die Duftkartusche.

In der vorstehend beschriebenen Ausführungsform, in der die Duftkartusche dem Filter nachgeschaltet ist, hat diese eine im Wesentlichen dreieckige Form, wobei zwei Seiten dem Lufteintritt und die dritte Seite dem Luftaustritt vorbehalten sind.

Es ist zweckmäßig, die Duftkartusche so auszustatten, dass der Luftdurchtritt gesteuert erfolgt. Insbesondere soll durch den gesteuerten Luftdurchtritt eine Aktivierung der Duftkartusche nur dann erfolgen, wenn das Fahrzeug in Gebrauch ist. Hier gibt es mehrere Möglichkeiten, die alternativ oder kumulativ eingesetzt werden können.

Zum einen kann die Duftkartusche an den diversen Öffnungen mit Folie verklebt sein, die segmentweise entfernt werden kann. In unbenutztem Zustand, vor dem Einbau in das erfindungsgemäße System kann damit der Verlust an Duftstoff verhindert werden. Nach Einbau kann durch segmentweises Entfernen der Folie von den Öffnungen das Ausmaß der Duftabgabe bestimmt werden.

Dieses im Wesentlichen passive System, bei dem auch im Ruhezustand des Fahrzeugs Duftstoff abgegeben wird, wird zum anderen durch ein aktives Steuerungssystem ergänzt, das eine Aktivierung der Duftkartusche nur im Betriebszustand des Fahrzeugs gewährleistet. Betriebszustand bedeutet, dass entweder die Lüftung eingeschaltet ist oder aber das Fahrzeug in Betrieb ist und die jeweils vorhandene Zwangslüftung einen minimalen Luftdurchsatz durch die Lüftung gewährleistet.

Erfindungsgemäß erfolgt eine Regelung und Steuerung der Duftabgabe über einen mit der Duftkartusche zusammenwirkenden Motor. Dazu weist das System an der Duftkartusche einen oder mehrere Schieber oder Klappen auf, die die Öffnungen für den Lufteintritt und/oder den Luftaustritt verschließen und/oder freigeben können. Die Schieber und/oder Klappen sind mit einem kleinen Motor gekoppelt, der den Schieber von einer Verschlussposition in eine Offen-Position und zurückbewegen kann. Auch Zwischenstellungen sollten möglich sein. Der Motor ist an die Kraftfahrzeugelektrik angeschlossen und über den Bordcomputer im Fahrzeug steuerbar. Entsprechendes gilt für ein Klappensystem, das über einen Motor geöffnet und geschlossen werden kann.

Die Regelung kann insbesondere auch über einen in die Kartusche selbst integrierten Mikroantrieb erfolgen. Solche Mikroantriebe sind bekannt, beispielsweise aus der Kameratechnik. Es versteht sich, dass die Kartusche zu diesem Zweck über eine Schnittstelle mit dem Bordcomputer und der Bordelektrik verbunden ist.

Die Erfindung betrifft weiterhin eine Duftkartusche für das oben beschriebene System zur Einbringung von Duftstoffen in den Innenraum von Kraftfahrzeugen, die dazu bestimmt ist, mit dem Luftfilter des Lüftungssystems verbunden zu werden, wobei die Duftkartusche wenigstens eine Lufteintrittsöffnung und wenigstens Luftaustrittsöffnung aufweist sowie einen Raum mit einen den Duftstoff abgebenden Substrat. Diese Duftkartusche weist Halteelemente zur Verbindung mit einem Luftfilter auf, vorzugsweise einen oder mehrere Steckverbinder, die einen. Widerhaken haben und durch den Luftfilter durchgesteckt werden können. Die Duftkartusche hat vorzugsweise eine dreieckige Gestalt, so dass sie formschlüssig in die Falten eines Faltenfilters eingepasst werden kann. Zweckmäßigerweise sind die Halteelemente auf der Lufteintrittsseite der Duftkartusche angeordnet.

Wie vorstehend dargelegt, kann die Duftkartusche einen Mikromotor integriert enthalten, der geeignet ist, das in der Duftkartusche vorhandene Schieber- oder Klappensystem zu betätigen und damit den Luftdurchtritt freizugeben, teilweise freizugeben oder zu blockieren. Ein solcher Mikromotor wird mit der Kartusche entsorgt. Es versteht sich, dass die entsprechenden elektrischen Anschlusssysteme ebenfalls vorhanden sind.

Für den Fall eines extern angeordneten Motors, weist die Duftkartusche entsprechende Kopplungssysteme auf, über die ein Schieber- oder Klappensystem von diesem externen Motor betätigt werden kann.

Die Erfindung betrifft weiterhin auch eine Duftkartusche, die in für Transport und Lagerzwecke luftdicht versiegelter Form vorliegt. Dazu kann sie beispielsweise mit Folie abgeklebt sein, die vor Ingebrauchnahme abgezogen wird, oder in einen Plastikbeutel eingeschweißt sein.

Die Duftkartuschen können in zahlreichen Duftvarianten zur Verfügung stehen und eingebaut werden. Es besteht auch die Möglichkeit, einen Filtereinsatz mit mehreren Duftkartuschen unterschiedlicher Duftvarianten auszustatten, von denen dann jeweils nur eine, je nach Präferenz, aktiviert wird.

Die Erfindung betrifft schließlich eine Kombination aus Duftkartusche und Luftfilter, insbesondere als Austauschelement in einbaufertiger Form.

Die Erfindung wird durch die Abbildungen näher erläutert. Es zeigen:
- Figur 1: einen Filter und eine Duftkartusche für die konstante Dosierung von Duftstoffen, die nicht der Erfindung entsprechen;
- Figur 2: eine erfindungsgemäße, motorgesteuerte Variante der Duftkartusche mit Filter; und
- Figur 3: die Duftkartusche von Figur 2 in aufgeschnittenem Zustand.

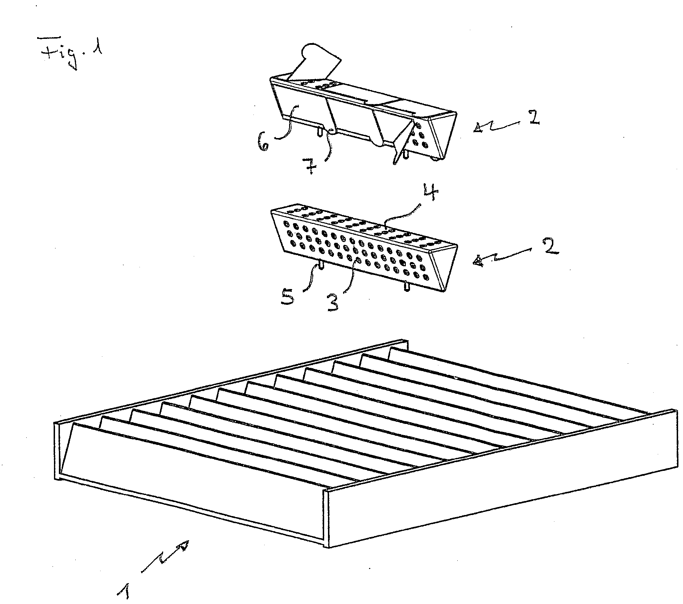

Figur 1 zeigt eine nicht erfindungsgemäße, einfache Variante des Systems mit einem herkömmlichen Faltenfilter 1 und einer Duftkartusche 2. Der Faltenfilter 1 besteht aus einem herkömmlichen Filtermaterial, das geeignet ist, Staub, Ruß, Pollen und andere unerwünschte Partikel aufzufangen. In der Regel besteht das Filterelement aus einem Faservlies, das in entsprechende Falten gelegt wird und in einen entsprechenden Einschub im Lüftungssystem eingepasst wird.

Die Duftkartusche 2 ist äußerlich an die Form des Faltenfilters angepasst, dergestalt, dass sie von ihren Konturen formschlüssig mit den Lufteingangsseiten an den Falten des Filters anliegt. Die Lufteingangsseiten zeigen Öffnungen 3, durch die die gefilterte Luft in die Kartusche eintreten kann. Zwei Steckverbinder 5 werden durch das Filtervlies durchgesteckt und halten die Duftkartusche in Position. Im Inneren der Duftkartusche 2 befindet sich ein Raum, der mit dem mit Duftstoff beladenen Substrat, vorzugsweise einem porösen polymeren Granulat, gefüllt ist. An der freien Seite befinden die Luftaustrittsöffnungen 4, durch die die mit Duftstoff beladene gefilterte Luft austritt.

Wie oberhalb der aktivierten Kartusche dargestellt, ist die Kartusche in unbenutztem Zustand im Bereich ihrer Eintritts- und Austrittsöffnung mit Folie abgeklebt. Im dargestellten Fall sind die Öffnungen mit einer Mehrzahl von Folien 6 verklebt, die alle eine Handhabungshilfe 7 aufweisen. Mit der Handhabungshilfe 7 können die Folien abgezogen werden, wobei es dem Benutzer überlassen bleibt, eine, mehrere oder alle Folien abzureißen und dadurch das Ausmaß der Duftbeladung der Frischluft zu bestimmen. Je größer der Luftdurchtritt, desto stärker die Beduftung.

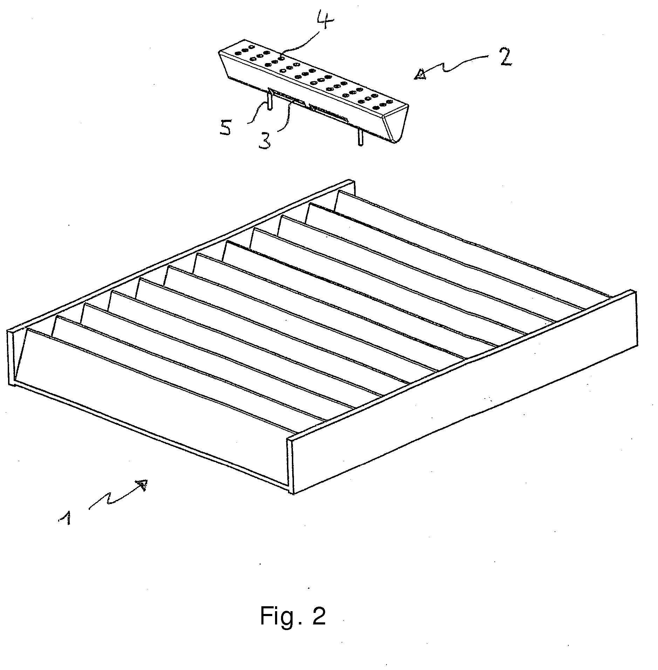

Figur 2 zeigt eine erfindungsgemäße Variante des Systems, bei dem die Kartusche 2 seitliche Lufteintrittsöffnungen 3 in Schlitzform aufweist und eine Vielzahl von Luftaustrittsöffnungen 4 an der Oberseite. Auch hier sind Steckverbinder 5 vorhanden.

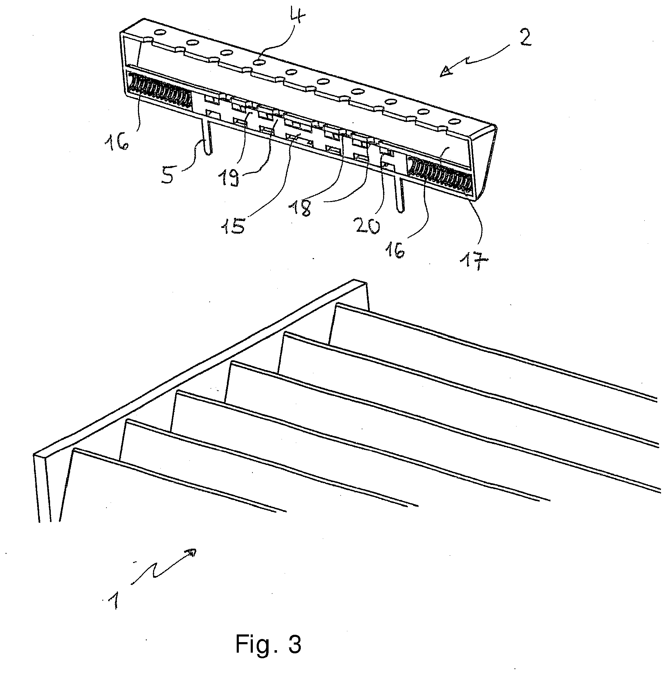

Die Funktionsweise der Duftkartusche von Figur 2 ergibt sich aus der Figur 3, die die Duftkartusche 2 in aufgestelltem Zustand zeigt. Im unteren Bereich der Kartusche 2 befindet sich ein Schiebersystem 15, das an beiden Seiten mit Federelementen 16 versehen ist. Die Federelemente 16 stützen sich an den stirnseitigen Wänden der Kartusche 2 ab.

Innerhalb der Kartusche 2 befindet ein Raum 17, in dem das mit Duftstoff beladene Granulat gelagert wird. Dieser Raum 17 hat im Bodenbereich Öffnungen 18, die mit Verschlusselementen 19 des Schiebers 15 korrespondieren. Im Ruhezustand des Schiebers 15 sind die Öffnungen 19 mit den Verschlusselementen 18 verschlossen. Wird allerdings der Schieber 15 in die eine oder andere Richtung verschoben gibt er über die zwischen den Verschlusselementen 19 liegenden Einschnitte 20 die Öffnungen 18 frei, so dass die Luft durch die seitlichen Eintrittsöffnungen 3 und die Öffnungen 18 in das Granulat eintreten und durch die Austrittsöffnung 4 in den Innenraum des Fahrzeugs austreten kann.

In der Ausführungsform von Figur 2/3 wird die Schieberfunktion erfindungsgemäß über einen Motor gesteuert. Der Schieber wird nach Vorgabe des Fahrers über die Bordelektronik durch den Motor bewegt. Ein solcher Motor ermöglicht den kompletten Verschluss der Duftkartusche wie auch eine vollständige Öffnungen und Zwischeneinstellungen. Der Motor kann ein in die Kartusche integrierter Mikromotor sein oder ein externer Motor, der über eine Kupplungsstange den Schieber bewegt.

## Patentansprüche

1. System zur Einbringung von Duftstoffen in den Innenraum eines Fahrzeugs über das Lüftungssystem mit einer Kombination aus einer Duftkartusche (2) und einem Luftfilter (1), wobei die Duftkartusche (2) wenigstens eine Lufteintrittsöffnung (3) und wenigstens eine Luftaustrittsöffnung (4) aufweist, sowie einen Raum (17) mit einem den Duftstoff abgebenden Substrat, wobei die Duftkartusche (2) am Luftfilter (1) so verankert ist, dass bei aktiviertem Lüftungssystem die Duftkartusche (2) von Luft durchströmt werden kann **dadurch gekennzeichnet, dass** die Duftkartusche (2) einen oder mehrere Schieber (15) oder Klappen aufweist, die mit einem Motor gekoppelt sind, und die die Öffnungen für den Lufteintritt (3) und/oder Luftaustritt (4) verschließen bzw. frei geben.

2. System nach Anspruch 1, **dadurch gekennzeichnet, dass** die Schieber oder Klappen beliebig zwischen einer geschlossenen und einer offenen Stellung positionierbar sind.

3. System nach Anspruch 1 oder 2, **dadurch gekennzeichnet, dass** der Motor ein Mikromotor ist.

4. System nach Anspruch 3, **dadurch gekennzeichnet, dass** der Mikromotor in die Duftkartusche integriert ist und an die Fahrzeugelektrik angeschlossen ist.

5. Duftkartusche für das System eines der Ansprüche 1 bis 4, mit wenigstens einer Lufteintrittsöffnung (3), wenigstens einer Luftaustrittsöffnung (4) und einem Raum (17), der ein einen Duftstoff abgebendes Substrat enthält, wobei sie eine Steckverbindung (5) zur Festlegung an einen Luftfilter (1) aufweist, **gekennzeichnet durch** einen Schieber (15) vor der oder den Lufteintrittsöffnungen (3).

6. Duftkartusche nach Anspruch 5, **dadurch gekennzeichnet, dass** sie auf der Lufteintrittsseite eine Dreiecksform zur Festlegung an einen Faltenfilter (1) aufweist.

7. Duftkartusche nach Anspruch 6, **dadurch gekennzeichnet, dass** die Schieber oder Klappen beliebig zwischen einer geschlossenen und einer offenen Stellung positionierbar sind.

8. Duftkartusche nach Anspruch 7, **dadurch gekennzeichnet, dass** die Schieber mit einem Motor gekoppelt werden können.

9. Duftkartusche nach Anspruch 8, **dadurch gekennzeichnet, dass** der Motor ein Mikromotor ist.

10. Duftkartusche nach Anspruch 9, **dadurch gekennzeichnet, dass** der Mikromotor in die Duftkartusche integriert ist und an die Fahrzeugelektrik angeschlossen ist.

## Claims

1. A system for introducing fragrances into the interior of a vehicle by way of the ventilation system with a combination of a fragrance cartridge (2) and an air filter (1), wherein the fragrance cartridge (2) has at least one air inlet opening (3) and at least one air outlet opening (4), and also a space (17) with a substrate which gives off the fragrance, wherein the fragrance cartridge (2) is anchored to the air filter (1) such that when the ventilation system is activated air can flow through the fragrance cartridge (2),
**characterised in that**
the fragrance cartridge (2) has one or more slides (15) or flaps which are coupled to a motor, and which close or uncover the openings for the air inlet (3) and/or air outlet (4).

2. A system according to Claim 1, **characterised in that** the slides or flaps may be positioned as desired between a closed and an open position.

3. A system according to Claim 1 or Claim 2, **characterised in that** the motor is a micromotor.

4. A system according to Claim 3, **characterised in that** the micromotor is integrated in the fragrance cartridge and is connected to the vehicle electrical system.

5. A fragrance cartridge for the system of one of Claims 1 to 4, with at least one air inlet opening (3), at least one air outlet opening (4) and a space (17) which contains a substrate which gives off a fragrance, it having a plug connection (5) for fixing to an air filter (1), **characterised by** a slide (15) in front of the air inlet opening or openings (3).

6. A fragrance cartridge according to Claim 5, **characterised in that** on the air inlet side it has a triangular shape for fastening on a pleated filter (1).

7. A fragrance cartridge according to Claim 6, **characterised in that** the slides or flaps may be positioned as desired between a closed and an open position.

8. A fragrance cartridge according to Claim 7, **characterised in that** the slides can be coupled with a motor.

9. A fragrance cartridge according to Claim 8, **characterised in that** the motor is a micromotor.

10. A fragrance cartridge according to Claim 9, **characterised in that** the micromotor is integrated in the fragrance cartridge and is connected to the vehicle electrical system.

## Revendications

1. Système d'introduction de substances odorantes dans l'habitacle d'un véhicule par le système d'aération comprenant une combinaison d'une cartouche odorante (2) et d'un filtre à air (1), la cartouche odorante (2) ayant au moins une ouverture d'entrée d'air (3) et au moins une ouverture de sortie d'air (4) ainsi qu'un espace (17) renfermant un substrat délivrant la surface odorante, la cartouche odorante (2) étant ancrée sur le filtre à air (1) de sorte que lorsque le système d'aération est activé, la cartouche odorante (2) puisse être traversée par de l'air,
**caractérisé en ce que**
la cartouche odorante (2) comprend au moins un tiroir (15) ou un volet qui est couplé à un moteur et qui ferme ou libère les ouvertures d'entrée d'air (3) et/ou de sortie d'air (4).

2. Système conforme à la revendication 1,
**caractérisé en ce que**
le tiroir ou le volet peut être sélectivement positionné entre une position fermée et une position ouverte.

3. Système conforme à la revendication 1 ou 2,
**caractérisé en ce que**
le moteur est un micromoteur.

4. Système conforme à la revendication 3,
**caractérisé en ce que**
le micromoteur est intégré dans la cartouche odorante et est connecté au système électronique du véhicule.

5. Cartouche odorante destinée à un système conforme à l'une des revendications 1 à 4,
comportant au moins une ouverture d'entrée d'air (3), au moins une ouverture de sortie d'air (4) et un espace (17) qui renferme un substrat délivrant une substance odorante, cette cartouche comprenant une connexion par fiche (5) permettant sa fixation à un filtre à air (1),
**caractérisée par**
un tiroir (15) installé avant la ou les ouverture(s) d'entrée d'air (3).

6. Cartouche odorante conforme à la revendication 5,
**caractérisée en ce qu'**
elle a, sur le côté d'entrée d'air une forme triangulaire permettant sa fixation à un filtre à plis (1).

7. Cartouche odorante conforme à la revendication 6,
**caractérisée en ce que**
le tiroir ou le volet peut être positionné sélectivement entre une position fermée et une position ouverte.

8. Cartouche odorante conforme à la revendication 7,
**caractérisée en ce que**
le tiroir peut être couplé à un moteur.

9. Cartouche odorante conforme à la revendication 8,
**caractérisée en ce que**
le moteur est un micromoteur.

10. Cartouche odorante conforme à la revendication 9,
**caractérisée en ce que**
le micromoteur est intégré dans la cartouche odorante et est connecté au système électronique du véhicule.
